(19) **Europäisches Patentamt**

**European Patent Office**

**Office européen des brevets**

(11) **EP 1 386 972 A1**

(12) **EUROPEAN PATENT APPLICATION**

(43) Date of publication:
**04.02.2004 Bulletin 2004/06**

(51) Int Cl.⁷: **C12Q 1/68**, G06F 19/00

(21) Application number: **02017317.5**

(22) Date of filing: **01.08.2002**

| | |
|---|---|
| (84) Designated Contracting States:<br>**AT BE BG CH CY CZ DE DK EE ES FI FR GB GR**<br>**IE IT LI LU MC NL PT SE SK TR**<br>Designated Extension States:<br>**AL LT LV MK RO SI**<br><br>(71) Applicant: **Noxxon Pharma AG**<br>**13355 Berlin (DE)**<br><br>(72) Inventors:<br>• **Kleinjung, Frank**<br>**10829 Berlin (DE)** | • **Röhl, Ingo**<br>**10589 Berlin (DE)**<br>• **Wientges, Jens**<br>**10551 Berlin (DE)**<br><br>(74) Representative: **Betten & Resch**<br>**Patentanwälte,**<br>**Theatinerstrasse 8**<br>**80333 München (DE)** |

(54) **Method and apparatus for designing a nucleic acid having a desired property**

(57)     A method for finding spiegelmers, wherein said method comprises:

representing a first generation of a population of a variety of sequences in a computer readable form; synthesizing said a first generation as a set of spiegelmers

measuring the fitness of each of said synthesized population members which respect to a certain function in connection with a target substance by an assay which is sensitive for said function;

representing said first generation population members and said respective corresponding measured fitness values in a computer readable form;

based on said measured fitness values, selecting pairs of members of said parent population and carrying out a computer implemented mating for said pairs in order to generate next-generation population members as child sequences based on said parent population members which are mated;

synthesizing the next generation set of spiegelmers based on said child sequences which have been generated from said computer implemented mating step;

measuring the fitness of said synthesized next-generation population members; repeating said step of computer implemented generation of the next-generation population sequences, said step of synthesizing the corresponding spiegelmers; and said step of measuring the corresponding fitness until one or more members of the next-generation population have a fitness which lies beyond a desired value.

| A | T |
|---|---|
| G | C |

| AA | AT | TA | TT |
|----|----|----|----|
| AG | AC | TG | TC |
| GA | GT | CA | CT |
| GG | GC | CG | CC |

Fig. 11

**Description**

Field of the invention

The present invention relates to a method and an apparatus for finding a spiegelmer which has a certain desired property, like e.g. the capability of binding to a certain target.

Background of the invention

Chemical, biological and pharmaceutical scientists often are confronted with the problem that they have to create a new substance which meets a certain chemical or biological requirement. Such a requirement may for example be the capability to act as a binder for a specific target such that the substance may block the biological function of the target and may thereby achieve a corresponding biological or medical effect. The capability to act in the desired manner may also be called "fitness".

In the past years, technologies have been established in order to use nucleic acids in a manner previously unanticipated. An essential part of some of the new DNA or RNA techniques is called *in vitro* selection or evolution. Thereby, DNA or RNA with desired properties may be obtained from a library of heterogeneous nucleic acid molecules by means of variation, selection and amplification. Nucleic acids with highly affine binding properties may be isolated by means of a cyclic process of the polymerase chain reaction (PCR), transcription, selective binding and reverse transcription. This method for selecting a nucleic acid ligand that specifically binds to a desired target is termed SELEX, an acronym for **S**ystematic **E**volution of **L**igands by **E**xponential enrichment. Examples for such in vitro selection or evolution methods have been provided for example by Tuerk and Gold, Science 249 (1990), 505-510; Berzal-Herranz et al., Genes & Development 6 (1992), 129-134; by Robertson and Joyce, Nature 344 (1990), 467-468 and e.g. by the US Patents 5,475,096; 5,670,637; 5,696,249.

Unfortunately, natural nucleic acids (DNA and RNA) are highly unstable in biological environments (i.e. serum). this disadvantage can be completely circumvented using the enantiomeric forms of nucleic acids: L-RNA and L-DNA (named Spiegelmers).

Since L-nucleic acids are not compatible with SELEX because of the enantio-specificity of the enzymes used for amplification, a "mirror-image" SELEX approach is employed. The first step is to select an aptamer against the enantiomeric form of the natural target. After trimming to the minimal binding motif the equivalent L-form of the aptamer, the Spiegelmer, is then synthesized and, because of the reciprocal chirality, this Spiegelmer binds with high affinity to the natural target. Mirror-image RNA ligands to adenosine and arginine as well as an enantiomeric DNA specific for vasopressin were identified by "mirror-image" SELEX and have been described previously (1, 6, 7).

Klussmann, S., Nolte, A., Bald, R., Erdmann, V.A. & Fürste, J.P. (1996) Nat Biotechnol 14,1112-5.

Nolte, A., Klussmann, S., Bald, R., Erdmann, V.A. & Fürste, J.P. (1996) Nat Biotechnol 14, 1116-9.

Williams, K.P., Liu, X.H., Schumacher, T.N., Lin, H.Y., Ausiello, D.A., Kim, P.S. & Bartel, D.P. (1997) Proc Natl Acad Sci USA 94, 11285-90.

Beside the advantage of high biological stability some disadvantages are coupled to the Spiegelmer-technology as described above.

The synthesis of the full enantiomeric target is often expensive or even not possible technically.

The SELEX process itself is relatively costly and time-consuming.

The selection criterion in the SELEX process is always binding. Other functions (i.e. effectiveness in cell culture, inhibition of an enzyme ...) cannot be introduced as a selection (or fitness) criterion.

Summary of the Invention

In view of the disadvantages coming along with the approach used so far, the present invention intends to provide and improve a method for finding spiegelmers that meet one or more defined criteria.

According to a first aspect the present invention provides a method for finding spiegelmers, wherein said method comprises: representing a first generation of a population of a variety of sequences in a computer readable form;

synthesizing said a first generation as a set of spiegelmers;

measuring the fitness of each of said synthesized population members which respect to a certain function in connection with a target substance by an assay which is sensitive for said function;

representing said first generation population members and said respective corresponding measured fitness values in a computer readable form;

based on said measured fitness values, selecting pairs of members of said parent population and carrying out a computer implemented mating for said pairs in order to generate next-generation population members based on said parent population members which are mated;

synthesizing the next generation set of spiegelmers which have been generated from said computer implemented mating step;

measuring the fitness of said synthesized next-generation population members; repeating said step of computer implemented generation of the next-generation population sequences, said step of synthesizing the spiegelmers; and said step of measuring the corresponding fitness until one or more members of the next-generation population have a fitness which lies beyond a desired value.

The aforementioned method has several significant advantages when compared with the prior art SELEX process using the double mirror approach for the generation of spiegelmers.

First of all, all steps which are carried out in vitro can be performed using the natural enantiomer of the target. This means that the costly and difficult step of synthesizing a mirror target is not necessary anymore. Rather, for the in vitro steps of the method of the invention the natural target can be used. This is particularly relevant when considering that the synthesizing capabilities are limited and therefore the classical double mirror approach can not be applied for targets which due to limitations of synthesis capabilities cannot be synthesized in the mirror world.

Furthermore, with the method of the present invention biological assays can be used for determining the fitness. In the classical approach the "determination" step was limited to a measurement of the binding capability. With the present invention, however, one can take advantage of the large variety of assays available for directly measuring the efficiency with respect to a certain desired function rather than merely measuring the binding capability which does not necessarily correlate with the desired function.

A further advantage lies in the fact that by e.g. using different assays there can be determined complex "fitness parameters" which may be defined as multifunctional property indicators. It thereby becomes possible to optimize a spiegelmer to even a variety of desired functions rather than merely to a certain binding activity.

Since some steps of the method, namely the selection of the pairs for the mating step and the mating step itself are carried out in or by a computer, the method of the present invention is inexpensive and fast when compared with the classical SELEX approach.

The only in vitro steps are the synthesizing of the spiegelmer population members an the measurement of a score for each of the population members which indicates the fitness of the individual member with respect to the desired property. There is used an evolutionary approach which consists in the selection of parents chosen from the first generation population members and then generating a child population based on the selected parents. For this purpose a computer implemented method is used.

It is readily apparent to the skilled person that especially an efficient mating operation is desired to improve the efficiency of the creation of the next generation.

Apart from an efficient mating another approach of improving the fitness of the next generation also consists in the suitable selection of parents. For example, the parent population members may be ranked according to their fitness, possibly then the ranking furthermore is weighted by some weighting function to get the weighted fitness, and then parents are randomly chosen according to the thus obtained weighted fitness. This will improve the fitness of the next generation assuming that parents which have a better fitness have a higher probability of creating children which have an even improved fitness.

According to a further aspect of the present invention, there is generated a next generation population based on a parent population the members of which have a sequence length of at least m. From the parent population there are selected two members as parents by the mating of which a child population member is to be generated. It is then determined for sequence motives the length of which is n < m how well they coincide with patterns in the parent sequences. This is carried out for each of the parent sequences and for each possible motive of the length n. This results in a motive significance value indicating for a certain motive how well it coincides with patterns in a certain one of the parents.

Based on the individual significance values for the motive there is then calculated a total motive significance value for each motive. Based on the motives for which the total significance value is comparatively high there is then created a child.

The foregoing approach is based on the assumption that individual motives the length of which is smaller than the total sequence length of the parent population members are responsible for the property which leads to a high fitness, such as a binding capability. If such a motive indeed is responsible for a relatively high fitness, then there is a comparatively high likelihood that it can be found in both parent members selected for the generation of the child member. And then, this results in a relatively high total motive significance value for such a motive.

In order to improve the probability that the parents chosen will produce a child having a good fitness, they may be selected from those parents for which the fitness is comparatively high. For this purpose the parent population may be ranked according to their fitness, and possibly the scores according to which they are ranked may be furthermore weighted by a certain weighting function.

The determination of the individual motive significance value according to one embodiment may comprise a check how many consecutive characters of a certain motive can be in the same manner found in one of the parents.

The individual significance value may then just be the number of consecutive characters found in the motive as well as in the parent population member.

Detailed description

In the following we will describe in detail how we can find a spiegelmer having a desired property.

The starting point is an initial population of nucleic acid sequences. This initial population is e.g. computer generated in a random manner by any random generator known to skilled persons. Starting from this initial population there is then carried out an evolutionary approach consisting of the selection of parent members according to their fitness, mating the parents to generate an offspring population, and repeating this process until one or more of the child population members have a desired fitness.

First of all, however, the computer represented population is synthesized as a set of spiegelmers. Then there is determined a fitness value using an assay for each member of this initial population. The thus determined fitness values are then assigned to their corresponding sequences in the computer implemented representation, e.g. by forming an array which contains the sequences, possibly identifiers for the sequences so that they can be looked up, and the fitness values determined for each sequence, respectively. This data can be inputted e.g. manually by an operator. Once the data has been entered into the computer representation of the population, then the next step consisting of the selction of parents, the mating, and the generation of an offspring sequence population may be carried out. These steps all are performed in the computer, and once the offspring population has been generated, then the corresponding set of spiegelmers again is synthesizied and the fitness is determined experimentally.

This process is then repeated iteratively until the set of spiegelmers synthesized based on a computer generated offspring population of sequences meets a certain criterion, i.e. that at least one or more members of the population have a good fitness value or a fitness value lying beyond a vertain treshold.

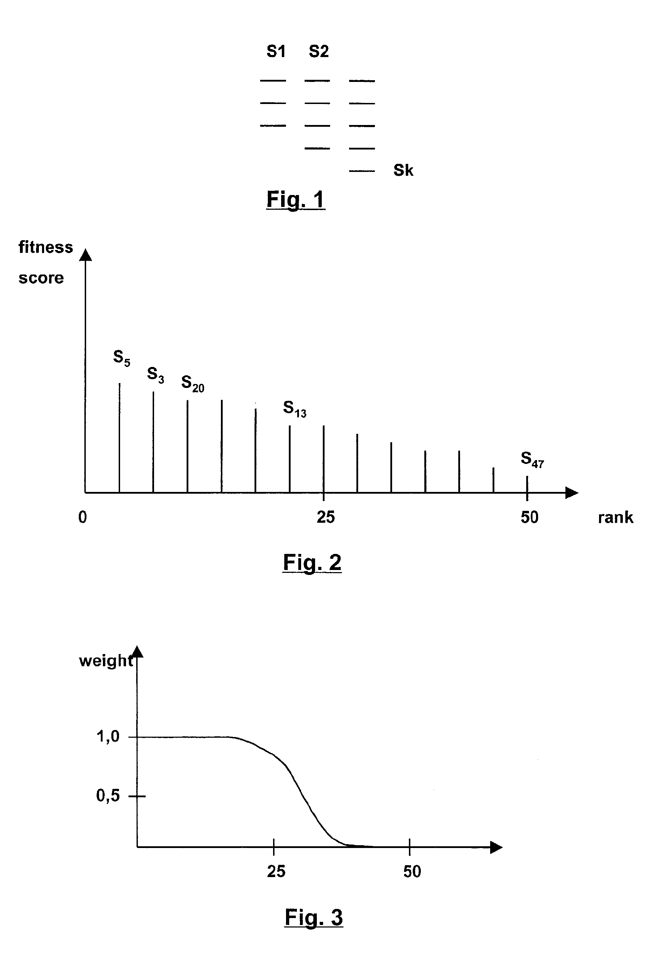

Assume that we have a parent population of sequences, for example of the length of 40 nucleic acids, which may be exemplarily shown in Fig. 1. There are in total k sequences, S1 to Sk. These sequences may be randomly generated and then act as the parent population. It will be readily apparent to the skilled person that the number of sequences and their length are to some extent arbitrary and may be chosen by the user depending on the synthesis capability and the computer power available.

There is exemplarily shown a set of parent members (a starting population) shown in Fig. 1. Each member may be represented in a computer by a cheracter sequence as it will be apparent to a person skilled in the art. Then the fitness is determined for each of these members. For that purpose the members of the parent population are synthesized in the form of non-natural enantiomers, i.e. for nucleic acids in their L-enantiomeric form. The fitness determination typically will then be determined experimentally, for example by experimentally checking how well the individual sequences act as a binder with respect to a certain target. For that purpose assays are commercially available which measure the desired property of the members of the parent population. Although it may in the future also be possible to determine the fitness computationally, the step of fitness determination at the present stage has to be carried out experimentally.

The fitness determination leads to a fitness score for each of the population members S1 to Sk. The members may then be ranked according to their fitness score as shown in Fig. 2. In Fig. 2 just for exemplary purposes sequence number 5 has the highest fitness score, and sequence number 47 has the lowest fitness score and is ranked last in the ranking of the x-axis. The ranking may be implemented in a computer by any sorting algorithm known to a person skilled in the art.

For the generation of the next generation now parents have to be selected from the parent population and the parents are then mated to create a child. The selection of parents may be carried out such that those sequences which have a higher fitness score may be preferably selected as parents for the creation of children.

For that purpose first of all the sequences or corresponding identifiers are stored in a computer together with their corresponding measured fitness scores. This can be done most easily e.g. in the form of a two-dimensional array. A computer program may then easily select the sequences which have e.g. the highest fitness, as will be readily apparent to a person skilled in the art.

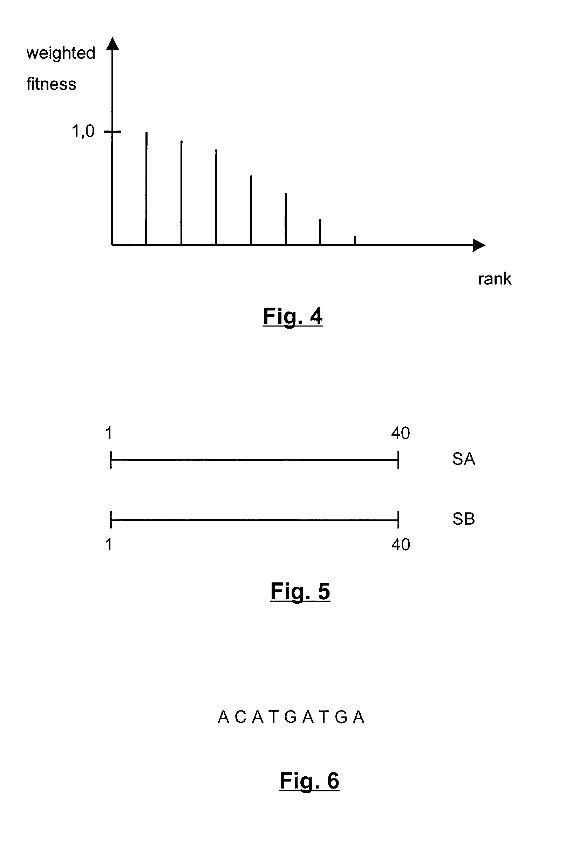

For the performance of the step of selecting the parents it may be useful to apply a weighting function such as shown in Fig. 3. In Fig. 3 a weight is shown for each rank and this weight is then multiplied with the fitness score of Fig. 2 to obtain a weighted fitness such as shown in Fig. 4. The parents may then be randomly selected but in a weighted fashion such that according to the weighted fitness the fitter members of the parent population are preferred in the random selection.

This may then result in the selection of two parent sequences SA and SB as shown in Fig. 5, each having a length of 40 characters (or nucleic acids). It should be clear that the length of 40 is only exemplarily chosen in the present embodiment, other sequence lengths are possible as well.

In the present example the sequences are sequences of nucleic acids, therefore each character in SA and SB (and in their computer representation) may either be G, A, C or T/U or any other nucleotide or any other modification of a nucleotide (for example at the base or at the sugar or the backbone). The computer representation of a sequence may either take the form of data stored in a volatile or a non-volatile memory, or it may be embodied in any computer-readable medium such as an electromagnetic wave or signal transmitted through a communications link like e.g. the internet.

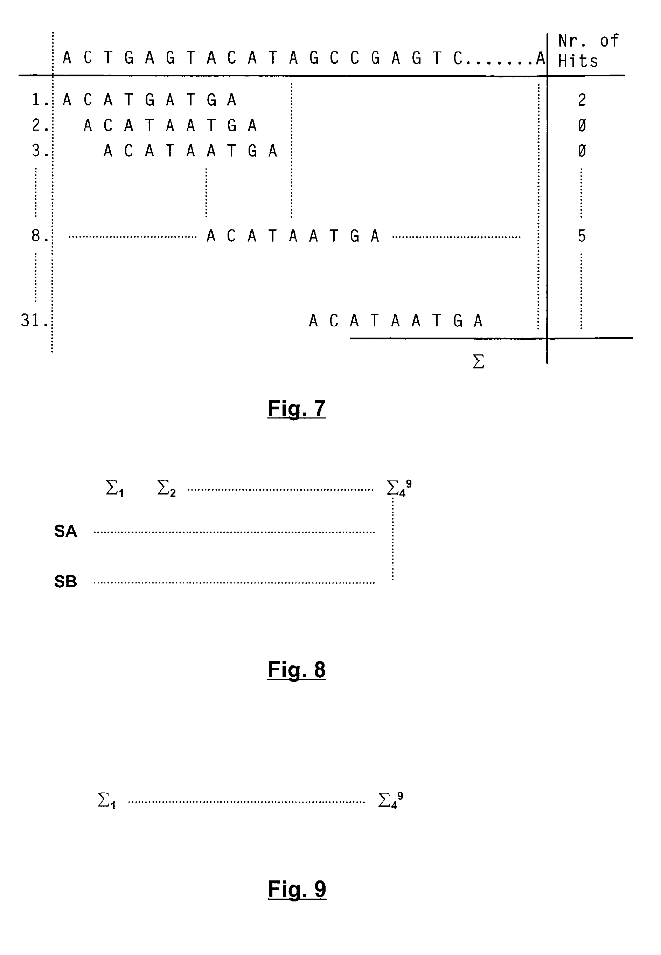

It is now in the next step checked which motives can be found in the individual sequences. For this purpose we use a motive sequence the length of which is smaller than the total sequence length of SA and SB. In the present exbodiment we chose a motive length of nine characters an example of which is shown in Fig. 6. If the motive length which is to be checked is nine characters as in the present example, then there are in total $4^9$ possible variations of this sequence motive as will be readily apparent to the skilled person. It is clear that the motive length may also be longer or shorter depending on the circumstances. The motive length should, however, be smaller than the sequence length.

For each of these $4^9$ possible variations or motives there is now carried out a check as to how well an individual motive sequence matches with fragments of the parent sequences SA and SB.

This is now in more detail explained in connection with Fig. 7. Fig. 7 is a table which in the heading row shows the sequence SA. In the next row numbered row (1) at the leftmost position there is indicated the motive shown in Fig. 6. It can be seen that the motive coincides in its first two characters with the sequence shown in the heading row. Therefore, in the rightmost column at the corresponding position the number of hits is indicated as 2.

Then in the next row the motive has been shifted one position to the right, and there is then again made a check as to how many characters coincide between the motive and the parent population member SA. In this case there is no hit. It should be mentioned that in the present embodiment as hits are only counted consecutive matches starting from the beginning character of the motive and not matches somewhere within the motive. For this reason the agreement between the last character A of the motive and the corresponding character in the parent sequence does not lead to a hit.

In row number 3 again the motive has been shifted one position to the right side, and again there is no hit. In the row number 8, however, in which the motive is shifted by seven characters to the right side, however, there is a considerable agreement between the motive sequence and the parent population sequence SA which results in a number of 5 hits as indicated in the rightmost column.

A comparison as explained before is then made for all starting positions of the motive by subsequently shifting the motive one character to the right hand side until the row number 31 of the table is reached. For each position of the motive the comparison with the parent sequence is made and the number of hits are counted and correspondingly indicated in the rightmost column.

Finally, the number of hits are added up to obtain a total sum of hits indicated as $\Sigma$ at the bottom of Fig. 7. This finally obtained total number of hits is an individual motive significance value which indicates how well the pattern of this special motive matches with the fragments of parent population member SA having the same length as said motive.

Such a check is now performed for all possible $4^9$ motives, and for all two parent population members.

Finally, there is thereby obtained a data structure as schematically illustrated in Fig. 8, namely an array of dimension 2 X $4^9$, and each member of this array indicates how well a certain motive is represented or can be found in a certain parent population member.

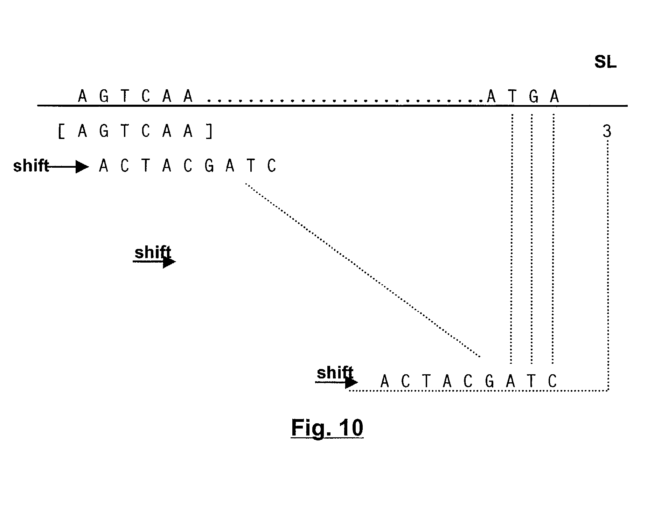

Based on these individual motive significance values there is then calculated a total significance value which gives an indication as to how well a certain motive is represented or can be found in both of said parent population members. This can in a most easy manner be done by calculating for example the sum of the individual motive significance values for each motive and for both parent population members such that for each motive there is then obtained a total motive significance value indicating how well this motive can be found in or matches with both of the parent population sequences. We therefore have finally obtained a vector as shown in Fig. 9, the vector having a dimension which equals the number of possible motives, namely $4^9$, and each element of the vector indicating for a certain motive how well it matches with both parent population members.

The next step now consists in the generation of a child population member based on the motives for which the total significance value is comparatively high. This will now be explained in detail by some specific examples.

The mating process generating offspring or next generation population members is now described in more detail. According to one embodiment it is composed of three steps:

1. represent the two sequences which are to be mated in a motive representation, which is an array of size 4^9, respectively
2. combine the two arrays to a combined parent representation
3. find a new sequence, the motive representation which is
4. as similar as possible to the combined parent-representation

In the following embodiment we assume that the population members are sequences which each have a length of 40 elements. The motives which are used in the present embodiment have the length 9. It will be readily apparent to the skilled person that these numbers may be changed and can be arbitrarily chosen according to the circumstances.

Each sequence is now represented by a so-called motive representation. The motive representation of a sequence indicates for each possible motive how well it matches with the sequence. For that purpose in the motive representation there exists a value for each possible motive, te so called individual motive significance value, and this value specific for a certain motive indicates how well this motive coincides with the sequence. The motive representation therefore can be implemented by an array of size 4^9, comprising a value for each 9-mer motive indicating how well this motive matches or coincides with said sequence or fragments thereof. As mentioned, this value can be called an individual motive significance value indicating how significantly a motive matches with a certain sequence.

A way of determining such an individual motive significance value according to a specific embodiment is now explained in connection with Fig. 10. The first row in Fig. 10 shows the sequence for which the motive significance value is to be determined. Below the sequence it is indicated how a motive for which the motive significance value is to be determined is shifted consecutively against the sequence. For each relative position between motive and sequence it is checked on how many positions there is a match between the sequence fragment and the motive. This number then may be called a location specific motive significance value $S_L$. The motive is then shifted along the sequence, for each relative position there is determined the location specific motive significance value, and the maximum number of the thus determined location specific motive significance values is then chosen to form the motive significance value for the given motive and the given sequence. This is schematically indicated in Fig. 10 in the rightmost column which contains the location specific motive significance values from which the maximum value is chosen to form the motive significance value for the given motive and the given sequence.

This process is also further explained in Table 1.1 which exemplarily shows how the comparison between the motives and the sequence fragments is made.

| Motive | | Motive set | |
|---|---|---|---|
| Gagtcgcac | | gagtcgcac | |
| | | gagatcgag | |
| | | gcgatacga | |
| **Subsequences** | **No. of matches** | **subsequences** | **No. of matches** |
| Gagtctgac | 7 | gagtctgac | 7 |
| Gagatcgac | 5 | gagatcgag | 9 |
| Atacgactc | 2 | atacgacta | 3 |

Table1.1: Some examples for determining the no. of matches, while the subsequences compared with this motive are shown in the lower boxes. The no. of matches indicates the number of positions where the subsequence and a motive are identical. The highest number of matches which is found when a motive is in a shifted manner compared with subsequences as described before then finally gives the motive significance value for this motive and the corresponding sequence.

It could also be imagined to determine the motive significance value in a different manner, e.g. such that only consecutive matches between motive and sequence may be allowed, then in case of the first subsequence in the left column of table 1.1 the identity number ( the location specific motive significance value) would be 6 instead of 7, because at the 7th position there is no match and the next match is only at position 9.

The determination of a motive significance value for each motive with respect to a certain sequence then results in a motive representation of each sequence, said motive representation indicating for a specific sequence and for all possible motives how well a certain motive matches or coincides with the specific sequence. It will be readily apparent to a skilled person that such a motive representation of a sequence cam be implemented be an array of the dimension 2 X (number of possible motives), while of course the number of the possible motives is 4^(motive length).

For each sequence of the population there is then generated such a motive representation.

The motive representation of a sequence may also be displayed graphically. For that purpose there may be used a mapping which maps each possible motive onto a position in the two-dimensional space, in other words, a pixel. The brightness of a pixel then corresponds to the motive significance value.

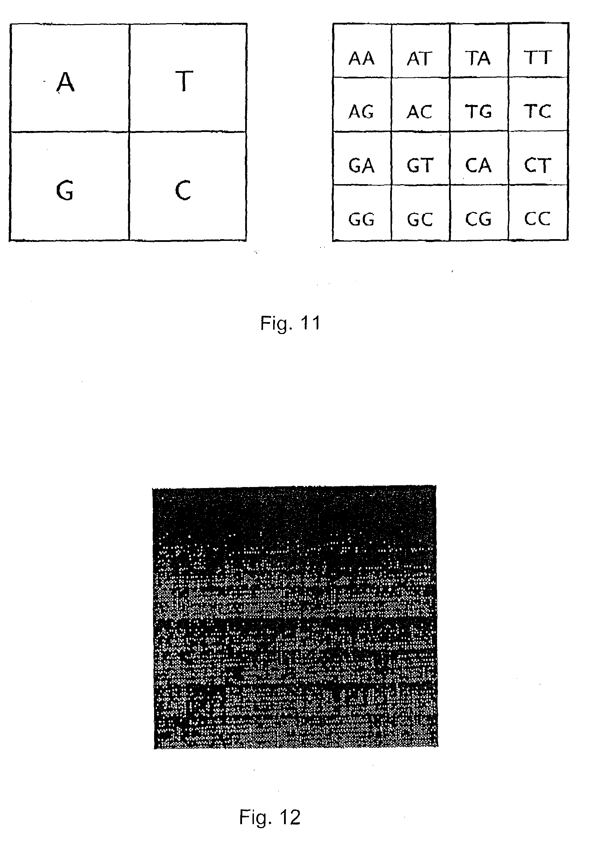

The generation of the graphical representation will now be explained in more detail in connection with Fig. 11.

Assume that we have the sequence elements A, T, G, and C. Let us further assume that we work with a motive length of 9. Then we need a graphical representation having 4^9 pixels, and each pixel must correspond in an identifiable manner to a certain one of the 4^9 possible motives.

Fig. 11 shows a square divided into four parts, the upper left represents 'A', the upper right 'T', the lower left 'G' and the lower right 'C'.

Each of these squares is now "expanded" as shown in the right-hand Figure of Fig. 11 by supplementing it with three additional squares, naming each with the character it had originally, and then appending to the original character either a A, G, T, or C depending on the position. Thereby the A of the left part of Fig. 11 becomes AA, AG, AT, and AC, the C of the left part for example becomes CA, CG, CA, and CC, respectively, and so on. The again each square is expanded by three additional squares, the original sequence is maintained in all for squares of the expanded square, and then an A, G, T, or, C is appended, respectively. In this manner finally an array of 4^9 pixels is generated where each pixel corresponds to one possible motive of the in total 4^9 motives.

Another way of describing the mentioned method of generating the graphical mapping is to quadruple each already existing square and then to append an A, T, C, and a G, repectively, to the squences already present in the squares. Repeating this procedure 9 times leads to a mapping between all possible 4^9 motives and a an array of 256^256 pixels.

The motive representation can then be graphically displayed as shown in Fig. 12. The brightness value may either be the motive significance value directly, or it may be a function thereof. Let the motive significance value be i, o being the length of the motive, and I being the length of the parent, then the brightness value v of a pixel of the motive representation may be calculated as follows:

$$v = -\log\left(1 - \left(1 - \frac{3^{o-l}\,o!}{i!\,(o-i)!}\right)^{l-n+1}\right)$$

This value is the negative logarithm of the probability to find this motive significance value by chance.

As mentioned before, there is generated a motive representation for each sequence of the population. The corresponding arrays may be stored in a computer or any computer readable medium together with a corresponding identifier, as will be readily apparent to a skilled person.

Based on the evaluation of the motive comparison there is then carried out the mating and the generation of the next generation population members. For that purpose first of all two sequences have to be selected which should act as parents and which should be mated. This can be done using the fitness ranking as already described before.

Once the two parents have been selected, then there is generated a combined motive representation which is based on the individual motive representation of the two parent sequences. For that purpose the two pictures are merged by combining the individual motive significance values for each pixel into a total motive significance value. The most easy way of doing so would be to just calculate the sum of the individual motive significance values for each pixel and using this sum as the total motive significance value in the merged motive representation. Other ways, however, can also be imagined. A preferable way of calculating the total motive significance value is the usage of the harmonic average as shown below:

$$v^* = \frac{2}{\frac{1}{va} + \frac{1}{vb}}$$

va here is the motive significance value or the brightness value for the first parent and vb the value for the second parent which have been already explained before.

It is, however, readily apparent to the skilled person that other ways of calculating the total motive significance value can be imagined as well.

Once the combined representation of the parents has been generated, a child sequence has to be created. In order to create the child sequence there is looked for a sequence which has will have a motive representation which has a high similarity to the combined motive representation of the parents.

The reasoning behind this approach is as follows. When selecting sequences having a high fitness as parents one has already made a selection which improves the likelihood that the next generation made from these parents has a good fitness. It is, however, most probably not the sequence as a whole which is responsible for the desired property but rather subsequences thereof, so called motives. By looking for such motives which match well with fragments of the sequence one can find motives for which the likelihood that they are responsible for the desired property is relatively high. This likelihood is even increased by searching for such motives which match well with both parent sequences, or in other words, which have a comparatively high total motive significance value.

The basic approach is now to find a child sequence the motive representation of which is similar to the motive representation of the parents. This leads to a comparatively high likelihood that the thus generated child has an even improved fitness.

To generate the child according to one embodiment the following steps are performed.

At first a sort algorithm sorts the motives ranked by their total motive significance values and selects e.g. the hundred best of them. In terms of the display representation this means that the hundred brightest pixels are selected.

Each of these pixels corresponds to a certain motive of the length $4^9$.

For each of the 100 motives the following step are performed.

The 9 bases of the motive form the first 9 bases of the child sequence. Then those four motives are search for which the first eight bases are identical to the last eight bases of the initial motive. For these four motives their respective total motive significance value is looked up, and the one for which this value is highest is selected. From the selected motive there is selected the last sequence element (the one which does not coincide with the initial motive), and this sequence element is chosen as the next sequence element of the child sequence (the 10th element). Then this process is repeated, i.e. those motives are searched for which the first eight sequence elements are identical to last 8 of the the meanwhile 10 sequence elements of the child sequence. Again, the last element of the one of the four motives having the highest total motive significance value is appended to the child sequence. In this manner the process is repeated until a child sequence of length 40 is obtained.

This process is carried out for all 100 motives which have been initially selected so that finally there are generated 100 child sequences.

For each of these child sequences there is now calculated a similarity measure to the combined motive representation of the parents, and the sequence for which this similarity measure is highest is then finally chosen as the child sequences.

A suitable similarity measure is e.g. the square deviation

$$s = \sum_{1}^{4^9} (va - vb)^2$$

Here va is the motive significance value of a certain motive for the child sequence, and vb is the total motive significance value of a certain motive for the combined parent motive representation. The lower s, the higher the similarity. Other similarity measures can however be used as well. Also, it is e.g. possible to choose any other number of initial motives than 100. One could e.g. also choose those motives for which the motive significance value lies beyond a certain treshold.

The process of generating a child sequence is repeated until the next generation population has been completely generated. Typically in each generation there is a certain defined number of population members, like in the present embodiment 40.

These 40 members which have been generated by the computer implemented method described before are then synthesized as spiegelmers and the fitness is then determined for the members of the next generation.

Again, based on the fitness values parents are selected and the computer implemented method for generating an offspring population is carried out which then again is synthesized as set of spiegelmers. This process is repeated iteratively until one or more members of a child generation have a good fitness.

In this manner it is possible to find spiegelmers which have a good fitness with respect to a desired property in a very efficient manner, i.e.quickly, inexpensively and with good fitness characteristics.

The aforementioned method has e.g. been employed to find a spiegelmer which has good binding properties with respect to ghrelin. This has some relevance because of the connections between ghrelin and obesity which are briefly explained in the following.

Obesity is the consequence of a sustained imbalance between energy intake and expenditure and is one of the leading health risks in the industrialized world. The causes for obesity are multi-factorial: they include socio-economical and psychological factors as well as genetic components. The molecular causes of the disease have been the subject of much research attention in recent years, greatly improving our understanding of how energy homeostasis is regulated. One outcome of these research activities has been the discovery of ghrelin, a 28 amino acid peptide hormone with an octanoyl acid side chain at the third serine of its N-terminus. This unusual modification is required for ghrelin's interaction at its natural target, the growth hormone secretagogue receptor 1a (GHSR1a). Ghrelin has been shown to mediate physiological functions pertinent to an anabolic state. While it directly stimulates the release of growth hormone (GH) from the pituitary, experiments in rodents also showed ghrelin to induce feeding in a GH-independent fashion by acting upon hypothalamic parts of the brain. Interestingly, the primary site of ghrelin production had been found to be in oxyntic glands in the stomach, suggesting that it serves as a hormonal link between stomach, pituitary and hypothalamus. Based on these findings ghrelin is thought to have a central role in the regulation of energy balance. The observation that ghrelin administration in rats resulted in weight gain as a consequence of changes in energy intake and/or fuel utilization is in support of such a role. Moreover, systemic ghrelin administration in humans caused sensations of hunger in the test subjects. Taken together, these data strongly implicate ghrelin as an important player in the regulation of an anabolic feeding state. (For background information see: M. Kojima, H. Hosoda, Y. Date, M. Nakazato, H. Matsu, K. Kangawa, "Ghrelin is a growth-hormone-releasing aylated peptide from stomach", Nature 402: 656-60, 1999; M. Tschöp, D.L. Smiley, M.L. Heiman, "Ghrelin induces adiposity in rodents", Nature 407:908-13, 2000; A.M. Wren et al., "Ghrelin enhances appetite and increases food intake in humans", Journal of Clinical Endocrinology Metabolism 86:5992-6, 2001; M. Nakazato et al., "A role for ghrelin in the central regulation of feeding", Nature 409: 194-8,2001.).

The development of a spiegelmer antagonist is therefore of strong scientific and pharmacological interest.

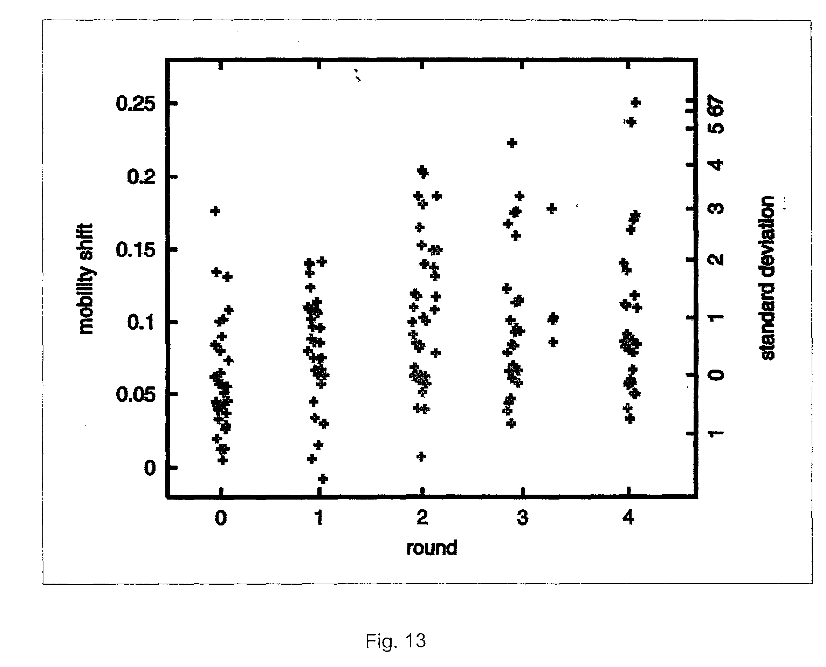

Using the aforementioned approach it was possible to find a spiegelmer which has very good binding properties with respect to ghrelin. In Fig. 13 the evolutionary steps of the process and the corresponding results are shown. The left-hand axis shows a measurement of the fitness, the right-hand axis shows $0^{th}$ to $7^{th}$ standard deviations as statistical measures indicating how likely (or better to say unlikely) it would be to find binders having such a fitness just randomly. Each point in the figure corresponds to a sequence, the population of round 0 has been randomly generated.

It can be seen that already after the $4^{th}$ evolutionary round already some sequences have been found for which the fitness is very good.

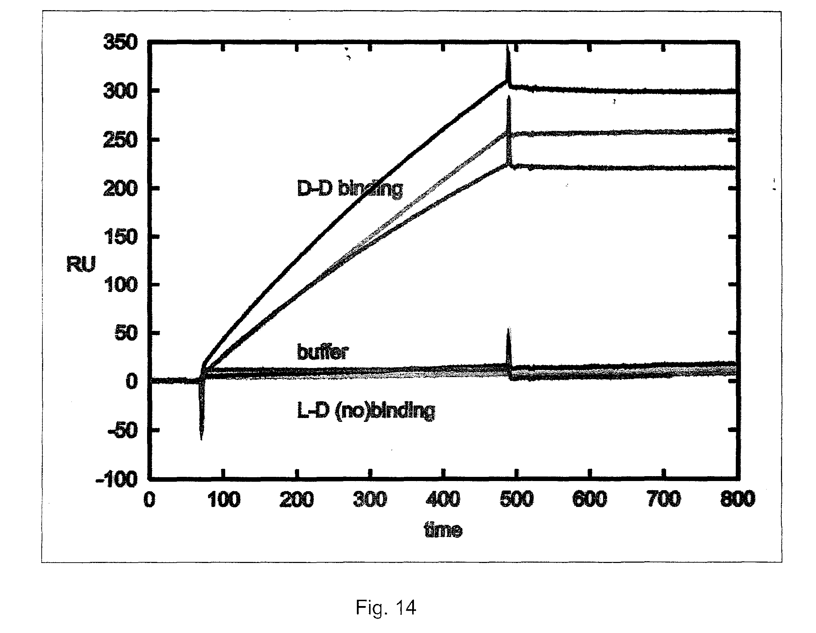

This result is confirmed by Fig. 14. Therein the y-axis shows the binding capability for the best 3 sequences of Fig. 13. It can be seen that they all are really very good binders.

Fig. 14 also shows that the binders indeed are specific with respect to chiral symmetry. It can be seen that only for the D-target and the d-nucleic binding sequence there are good binding properties, for the L-D pair there is no good binding efficiency.

It should be mentioned that for some practical purposes the selectivity of the binding has been shown by using a d-target instead of the natural L-target, however, the negative result for the L-D combination shown in Fig. 14 indeed proves that the found binder is specific for a certain enantiomeric symmetry.

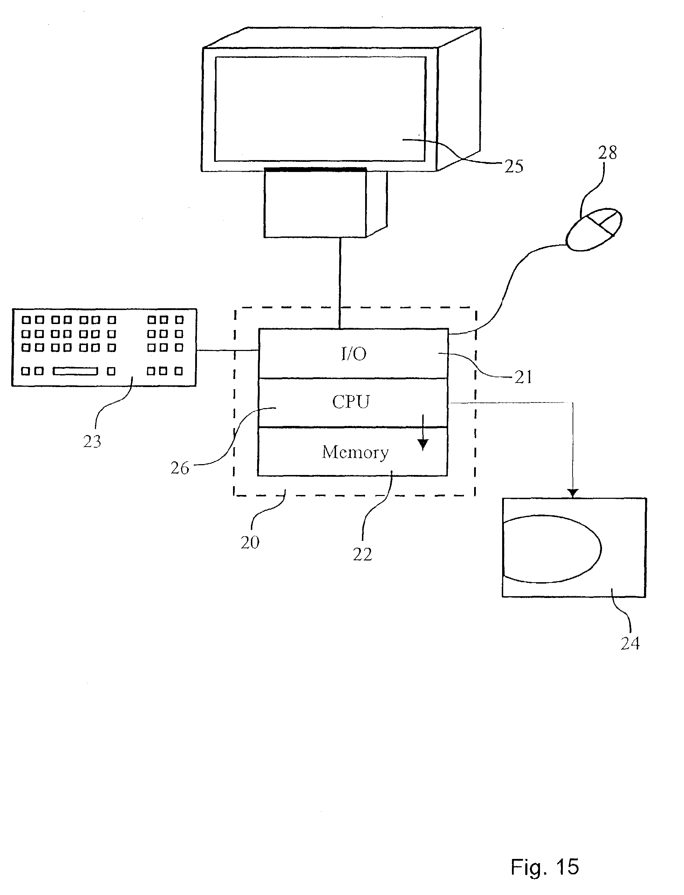

The computer-implemented steps may be performed by means of a standard computer as it is exemplarily shown in Fig. 15. The present invention is applicable to a hardware configuration like a personal computer or work station as illustrated schematically in Figure 14. The computer may comprise a central processing unit CPU 26, an input output IO unit 21, an internal memory 22 and an external memory 24. The computer further comprises standard input devices like a keyboard 23, a mouse 28 or speech processing means (not illustrated).

The computer implemented steps of the invention may be performed by running a computer program comprising program code on the computer of Fig. 15. The computer program code may be embodied in any form of a computer readable medium. The computer readable medium includes a storage medium as for example a RAM memory, a computer hard disc, an optical, magneto-optical or magnetic disc, card or tape and as well as a transport medium with and without employing carrier waves.

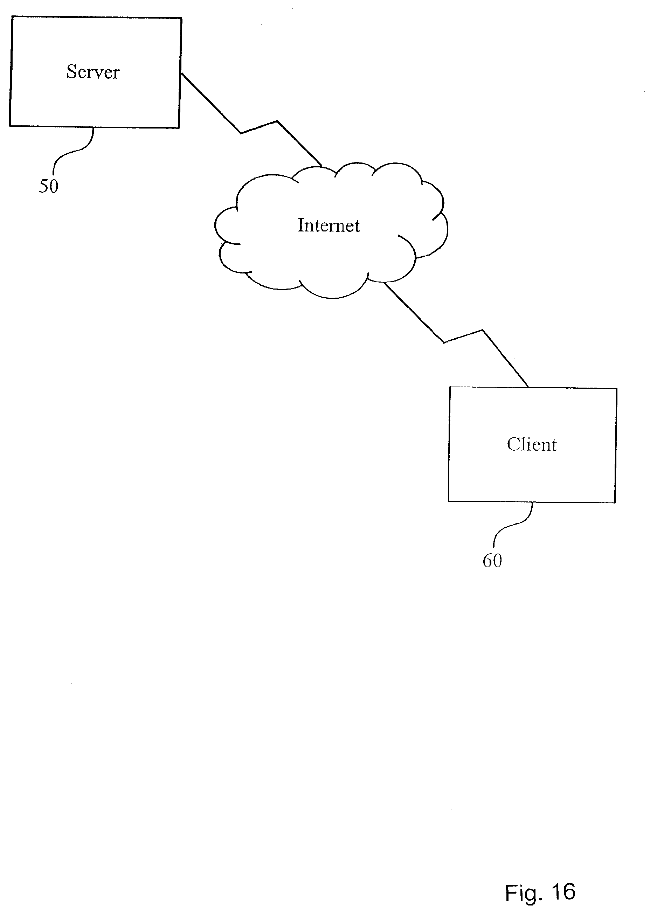

The invention, however, may also be applied to a client-server configuration as illustrated in Figure 16. The objects may be displayed on a display screen of a client device 60 while a number or all steps of the method as illustrated before in Figures 1 to 4 are carried out on a server computer 50 accessible by the client device 60 over a data network as the internet using a browser application or the like.

While the invention has been particularly shown with reference to a preferred embodiment thereof, it will be understood by those skilled in the art that various other changes in the form and details may be made therein without departing from the spirit and scope of the invention.

**Claims**

1.  A method for finding spiegelmers, wherein said method comprises:

representing a first generation of a population of a variety of sequences in a computer readable form;
synthesizing said a first generation as a set of spiegelmers;

measuring the fitness of each of said synthesized population members which respect to a certain function in connection with a target substance by an assay which is sensitive for said function;

representing said first generation population members and said respective corresponding measured fitness values in a computer readable form;

based on said measured fitness values, selecting pairs of members of said parent population and carrying out a computer implemented mating for said pairs in order to generate next-generation population members as child sequences based on said parent population members which are mated;

synthesizing the next generation set of spiegelmers based on said child sequences which have been generated from said computer implemented mating step;

measuring the fitness of said synthesized next-generation population members;

repeating said step of computer implemented generation of the next-generation population sequences, said step of synthesizing the corresponding spiegelmers, and said step of measuring the corresponding fitness until one or more members of the next-generation population have a fitness which lies beyond a desired value.

2. The method of claim 1, further comprising:

generating, based on a first generation, a second generation of a population of biological sequences of length m which in average has an improved fitness with respect to a certain biological property when compared to said first population, whereas each sequence of said first population is represented in a computer-readable medium as a character string, said method comprising:

selecting two members of said parent population having a good fitness as parents based on which a child is to be generated as a member of said second population, said generation of said child comprising:

for each individual parent sequence and for all possible motives, a motive being a sequence of length n < m, determining an individual motive significance value indicating to which extent a certain motive matches with a part of said parent sequence to thereby obtain a motive representation for each of said parent sequences;

calculating for each motive a total motive significance value based on said individual motive significance values of the parent motive representations, said total motive significance value thus being calculated forming a combined motive representation;

generating a new child sequence based on those motives for which the total motive significance value is high.

3. The method of claim 1or 2, wherein said step of selecting and mating comprises:

generating a motive representation representing each of the two sequences selected as a pair for mating by a corresponding array, said arrays respectively comprising a motive significance value for each motive indicating how well said motive matches with said sequence;

generating a combined motive representation by generating a combined array based on said pair of arrays, said combined array indicating for each motive a total motive significance value indicating how well a certain motive coincides with both parent sequences;

generating a new sequence such that the motive representation of the newly generated sequence is as similar as possible to the combined motive representation.

4. The method according to one of the preceding claims, wherein the total motive significance value is the harmonic average of said individual motive significance values for said two parents.

5. The method of one of the preceding claims, further comprising:

selecting a set of motives for which the total motive significance value is high;

generating a child sequence based on each of said motives of said set;

measuring the similarity between the thus generated child sequences and the combined motive representation;

selecting the child sequence having the highest similarity as the final child sequence.

6. The method of one of the preceding claims, wherein said generation of a child sequence comprises:

selecting as an initial motive a motive for which the total motive significance value is comparatively high;

determining the total motive significance values for those motives for which the first n sequence elements coincide with the last k sequence elements of the initial motive, with n being a number smaller than the motive length k;

appending the last k-n sequence elements of the motive for which the total motive significance value is highest to the initial motive to generate an enlarged motive;

using said enlarged motive as initial motive and repeating said steps of determining and appending until the enlarged motive has the length of the desired child sequence.

7. The method of claim 6, further comprising:

performing said generation of a child sequence for a plurality of initial motives to obtain a plurality of child sequences;

determining a similarity measure for each of said generated child sequences which indicates how similar said child sequences are to the combined motive representation of the parents from which the child sequences are generated;

selecting the child sequence with the highest similarity measure as the final child sequence.

8. The method of claim 6 or 7, wherein (k-n)=1 so that in each appending step the initial motive is enlarged by one further sequence element.

9. Spiegelmer, obtainable by a method according to claims 1 to 8.

10. The Spiegelmer according to claim 9, wherein said spiegelmer is a binder for the target ghrelin.

11. An apparatus for finding spiegelmers, wherein said apparatus comprises:

means for representing a first generation of a population of a variety of sequences in a computer readable form;

means for synthesizing said a first generation as a set of spiegelmers

means for measuring the fitness of each of said synthesized population members which respect to a certain function in connection with a target substance by an assay which is sensitive for said function;

means for representing said first generation population members and said respective corresponding measured fitness values in a computer readable form;

means for based on said measured fitness values, selecting pairs of members of said parent population and carrying out a computer implemented mating for said pairs in order to generate next-generation population members as child sequences based on said parent population members which are mated;

synthesizing the next generation set of spiegelmers based on said child sequences which have been generated from said computer implemented mating step;

measuring the fitness of said synthesized next-generation population members; repeating said step of computer implemented generation of the next-generation population sequences, said step of synthesizing the corresponding spiegelmers; and said step of measuring the corresponding fitness until one or more members of the next-generation population have a fitness which lies beyond a desired value.

12. The apparatus of claim 11, further comprising:

means for performing the method according to one of claims 2 to 8.

13. A Computer program comprising computer readable code for enabling a computer to carry out the computer implemented steps of one of claims 1 to 8.

S1    S2

——    ——    ——
——    ——    ——
——    ——    ——
——    ——
—— Sk

## Fig. 1

fitness

score

$S_5$

$S_3$  $S_{20}$

$S_{13}$

$S_{47}$

0              25              50              rank

## Fig. 2

weight

1,0

0,5

25              50

## Fig. 3

**Fig. 4**

**Fig. 5**

A C A T G A T G A

**Fig. 6**

| | A C T G A G T A C A T A G C C G A G T C . . . . . . . A | Nr. of Hits |
|---|---|---|
| 1. | A C A T G A T G A | 2 |
| 2. |   A C A T A A T G A | Ø |
| 3. |     A C A T A A T G A | Ø |
| 8. | A C A T A A T G A | 5 |
| 31. | A C A T A A T G A | |
| | $\Sigma$ | |

**Fig. 7**

$\Sigma_1$    $\Sigma_2$ ———————————— $\Sigma_4{}^9$

SA ————————————

SB ————————————

**Fig. 8**

$\Sigma_1$ ———————————— $\Sigma_4{}^9$

**Fig. 9**

SL

A G T C A A ............................. A T G A

[ A G T C A A ]

shift⟶ A C T A C G A T C

3

shift

shift    A C T A C G A T C

**Fig. 10**

| A | T |
|---|---|
| G | C |

| AA | AT | TA | TT |
|----|----|----|----|
| AG | AC | TG | TC |
| GA | GT | CA | CT |
| GG | GC | CG | CC |

Fig. 11

Fig. 12

Fig. 13

Fig. 14

I/O — 21

CPU

26

Memory

23

20

22

25

28

24

Fig. 15

Server
50

Internet

Client
60

Fig. 16

**European Patent Office**

## PARTIAL EUROPEAN SEARCH REPORT

which under Rule 45 of the European Patent Convention shall be considered, for the purposes of subsequent proceedings, as the European search report

Application Number

EP 02 01 7317

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 08856 A (BALD ROLF ;ERDMANN VOLKER A (DE); FUERSTE JENS PETER (DE)) 5 March 1998 (1998-03-05) * claims 1,23 * | 11,12 | C12Q1/68 G06F19/00 |
| X | WO 97 43444 A (SMITHKLINE BEECHAM PLC ;SMITHKLINE BEECHAM CORP (US); METCALF BRIA) 20 November 1997 (1997-11-20) | 11,12 | |
| A | * example 1 * | 1-8 | |
| A | FAMULOK M ET AL: "APTAMERS AS TOOLS IN MOLECULE BIOLOGY AND IMMUNOLOGY" CURRENT TOPICS IN MICROBIOLOGY AND IMMUNOLOGY, SPRINGER, BERLIN,, DE, vol. 243, 1999, pages 123-136, XP002952168 ISSN: 0070-217X * page 124 * | 1-8 | |

| | TECHNICAL FIELDS SEARCHED (Int.Cl.7) |
|---|---|
| | C12Q G06F |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 14 January 2003 | Gabriels, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**INCOMPLETE SEARCH
SHEET C**

Application Number

EP 02 01 7317

Claim(s) not searched:
      13

Reason for the limitation of the search (non-patentable invention(s)):

Article 52 (2)(c) EPC - Program for computers

                    --------------------

Further limitation of the search

Claim(s) not searched:
      9,10

Reason for the limitation of the search:

Present claims 9 and 10 relate to a spiegelmer defined by reference to a desirable characteristic or property, namely being a binder for the target ghrelin.

These claims cover all spiegelmers having this characteristic or property, whereas the application provides support within the meaning of Article 84 EPC and/or disclosure within the meaning of Article 83 EPC for none of such spiegelmers. In the present case, the claim so lacks support, and the application so lacks disclosure, that a meaningful search over the claimed scope is impossible. Independent of the above reasoning, the claims also lack clarity (Article 84 EPC). An attempt is made to define the spiegelmers by reference to a result to be achieved. Again, this lack of clarity in the present case is such as to render a meaningful search over the claimed scope impossible. Consequently, no search has been carried out for claims 9 and 10.

**EP 1 386 972 A1**

### ANNEX TO THE EUROPEAN SEARCH REPORT
### ON EUROPEAN PATENT APPLICATION NO.

EP 02 01 7317

14-01-2003

| Patent document cited in search report | Publication date | Patent family member(s) | Publication date |
|---|---|---|---|
| WO 9808856 A | 05-03-1998 | AT 228529 T | 15-12-2002 |
| | | AU 743469 B2 | 24-01-2002 |
| | | AU 4456897 A | 19-03-1998 |
| | | DE 59708838 D1 | 09-01-2003 |
| | | WO 9808856 A2 | 05-03-1998 |
| | | EP 0934331 A2 | 11-08-1999 |
| | | JP 2001504448 T | 03-04-2001 |
| WO 9743444 A | 20-11-1997 | EP 0928341 A1 | 14-07-1999 |
| | | WO 9743444 A1 | 20-11-1997 |
| | | JP 2000510699 T | 22-08-2000 |
| | | US 2001007743 A1 | 12-07-2001 |

EPO FORM P0459